(19) Europäisches Patentamt
European Patent Office
Office européen des brevets

(11) EP 1 322 167 B1

(12) EUROPEAN PATENT SPECIFICATION

(45) Date of publication and mention of the grant of the patent:
**02.11.2006 Bulletin 2006/44**

(21) Application number: **00967196.7**

(22) Date of filing: **29.09.2000**

(51) Int Cl.:
***A01N 59/16*** *(2006.01)*

(86) International application number:
**PCT/US2000/027019**

(87) International publication number:
**WO 2002/028187 (11.04.2002 Gazette 2002/15)**

(54) **ALLERGEN NEUTRALIZATION COMPOSITIONS**

ZUSAMMENSETZUNGEN ZUM NEUTRALISIEREN VON ALLERGENEN

COMPOSITIONS DE NEUTRALISATION D'ALLERGENE

(84) Designated Contracting States:
**DE ES FR GB IT**

(43) Date of publication of application:
**02.07.2003 Bulletin 2003/27**

(73) Proprietor: **THE PROCTER & GAMBLE COMPANY**
**Cincinnati, Ohio 45202 (US)**

(72) Inventors:
- **HASAN, Abul Khaer Mohamad,Quamrul**
  **Kobe 658-0032 (JP)**
- **MAO, Mark Hsiang-Kuen**
  **California 95758 (US)**
- **KOBAYASHI, Ryoko**
  **Kobe 658-0041 (JP)**

(74) Representative: **Veronese, Pancrazio et al**
**Procter & Gamble Italia S.p.A.**
**Italian Research Center**
**Via Aterno 92/94**
**66020 Sambuceto di San Giovanni Teatino**
**(Chieti) (IT)**

(56) References cited:
**WO-A-00/38626**
**WO-A-01/15712**
**WO-A-99/15208**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 322 167 B1

## Description

## TECHNICAL FIELD

The present invention relates to aqueous allergen neutralizing compositions comprising solubilized metal ions, and methods of using these compositions. The present compositions are particularly effective against the allergens associated with house dust mites and other common allergens such as cat dander, pollen and the like.

## BACKGROUND OF THE INVENTION

Sensitivity to allergens is a problem for an increasing number of consumers. This issue has been complicated by a surprising increase in asthma over the past few years. Asthma suffers are especially sensitive to airborne allergens. Allergy rates are also on the rise. This gives rise to increased awareness of the causes of allergy symptoms and how to decrease the associated discomfort.

Allergic reactions can be initiated in many ways, but one of the most common ways is by the inhalation of airborne allergens. Another common way to ingest allergens is when they come in direct contact with a moist surface on the body where they stick and react with the surrounding body tissue. This can happen, for example, when a person lays down in bed and allergen containing dust that lay on the pillow or bed coverings contacts and is absorbed into the person's eyes. This typically results in an allergic reaction as the allergens are gradually absorbed into the mucous around the eye.

Many allergens are protein based molecules, and these protein allergens can originate from many sources. It has been know for some time that one of the most common sources of allergens in a house is from dust mites. Of course, as is the case with all allergens, only certain people are allergic to dust mite allergens. But this group of people can be quite large in many areas, especially in hot humid areas. For example, in the south eastern United States of America, where it is both hot and humid for much of the year, the incidence of house dust mite allergies in the general population can be as high as 25%.

House dust mites thrive in plush carpets, overstuffed upholstery, cushy bed comforters and the like. Medical professionals who specialize in the treatment of allergies often recommend the removal of these items from the homes of people who suffer from dust mite allergies. For many reasons, this is often an impractical and unworkable solution to the problem of dust mite allergies.

Another common method for removing mite allergens from a house is the use of miteicides to kill the mites. But, unfortunately, the proteins that cause allergic reactions can be found in the mite corpses and also the residual mite feces. Thus, killing the mites does not solve the problem. And because some miticides can be relatively harsh chemicals, it might not be advisable to use these chemicals throughout your home on a regular basis.

Other methods for neutralizing allergens in the home include spraying household surfaces with compositions that either denature the allergens or simply cover them. The denaturing sprays, in theory, render the protein molecules inactive with respect to causing an allergic reaction. This is accomplished by chemical complexing between the allergen protein and the denaturing chemical such that the allergen can no longer initiate an allergic reaction in a human. Moreover, sprays can be used to "cover" or entrap the allergen contain particles. In theory, the "covered" allergens become too heavy to float in the air and cannot be inhaled. Likewise they do not directly contact the human body and cannot cause an allergic reaction. For various reasons, neither of these two approaches have proven entirely satisfactory for allergen sufferers.

Specifically, currently available allergen neutralization sprays typically use a tannin, or other related polyphenols, as the active component. But tannins and related chemicals are notorious for their strong yellow color. Thus, these chemicals cannot be sprayed onto fabrics without a substantial risk of staining the fabric material. As discussed above, fabrics such as bed covers, furniture covers, floor coverings, draperies, etc. are typically the areas where allergens can be found. Thus, few consumers feel comfortable spraying allergen neutralization compounds on their household fabrics.

WO 99/15206 describes for example deactivant compositions for dust mite allergens which comprise a great deal of deactivant compounds.

Likewise, the materials used to "cover" or encase allergen containing materials, such as dust, tend to be generally undesirable. Specifically, these materials typically "stick" to dust and other airborne particles making them too heavy to remain aloft. Unfortunately, both the spray and the covered particles become "sticky" resulting in fabric surfaces that feel sticky. This is especially undesirable for bed covers and pillows because they come into direct contact with the consumer's face. Moreover, it is well known that spraying a fabric with a sticky material results in attracting more dust and other airborne particles to the surface of the material. Ultimately, the surface becomes dirty much quicker that would normally occur.

For these reasons there exists a need for products that can neutralize allergens or at least keep them out of the air. These products should be sprayable and compatible with a wide variety of fabric materials. That is, the spray

products should not stain or otherwise damage common household fabrics that are used in making draperies, bed covers, carpeting, rugs, clothing, furniture covers and others. Moreover, these materials should create no additional health or environmental problems. For example, the spray products should not have a noxious smell nor should they be toxic to humans and household pets.

Importantly, and in addition to the needs discussed above, there exists a need for allergen neutralization products that have improved denaturing efficacy as compared to currently existing spray products. That is, the spray should denature a greater amount of proteins and a greater variety of proteins than currently available sprays. These and other needs are met by the allergen neutralization spray products of the present invention.

## SUMMARY OF THE INVENTION

In one aspect, the present invention comprises an allergen neutralization composition for use on inanimate objects. This composition comprises an effective amount of an allergy neutralizing metal ion, a solvent, and an allergen denaturing compound selected from the group consisting of polyphenol compounds, hydrogen peroxide, salicylic acid, citric acid, lactic acid, glycolic acid, ascorbic acid, gallic acid, gluconic acids, and mixtures thereof.

The metal ion is selected from the group consisting of ions of zinc, stannous, stannic, magnesium, calcium, manganese, titanium, iron, copper, nickel, and mixtures thereof. The allergen neutralization compositions of the present invention are sprayable. Preferably, the allergen neutralization compositions further comprise a wetting agent.

In one aspect of this invention the metal ion is present in the composition at about 0.01% to about 20%, preferably from about 0.1% to about 10%, more preferably from about 0.2% to about 8%, by weight, of the allergen neutralization composition.

The solvent for the allergen neutralization compositions of the present invention preferably comprises water, and more preferably the solvent comprises from about 0.01% to about 20%, by weight of the allergen neutralization composition, of a volatile lower alcohol. Even more preferably, the allergen neutralization compositions are essentially free of complexing agents. For example, cyclodextrin based complexing agents might bind with the metal ions or the additional allergen neutralization compounds which would decrease the available concentration of these active ingredients. By "essentially free of" it is meant the allergen neutralization composition contains less than about 0.5%, preferably less than about 0.1%, and even more preferably less than 0.01%, by weight, of complexing agents.

In another aspect of the present invention the allergen neutralization compositions further comprise at least about 0.05% by weight of an allergen neutralizing polyphenol compound, wherein the allergen neutralization composition yields a $\Delta E$ value of less than about 1.0 as measured by Fabric Color Evaluation method.

The allergen neutralization compositions of the present invention provide superior performance is reducing consumers allergy symptoms. These compositions operate on the principle of neutralizing the proteins associated with common house dust mites. Unfortunately, these proteins are not just associated with the live mites, but can be found in the feces and corpses of the house dust mites. The primary function of these allergen neutralization compositions is not to kill the house dust mites, but rather to neutralize the allergen proteins. The proteins can be neutralized chemically by denaturing, or they can be physically disabled by dust control methods. In either event, the proteins that cause allergic reactions in humans are neutralized or kept from entering the human body, as opposed to simply killing the mites.

The compositions of the present invention, in addition to providing improved efficacy, are compatible with a wide variety of household surfaces where house dust mites, and their feces and corpses might reside. This provides a substantial benefit because many known allergen neutralization compositions are known to discolor fabrics and hard surfaces. As can be appreciated, a product that is effective against allergen containing compounds will not be received favorably by consumers if it discolors or destroys the household surfaces on which it is used. Thus, the present compositions provide many unexpected and superior benefits over known allergen neutralizing compositions.

## DETAILED DESCRIPTION OF THE INVENTION

As used herein, the phrase "allergen neutralization" is intended to include both chemical denaturing and physical covering of the allergen containing protein. The compositions of this invention may neutralize allergens by chemically denaturing them, physically covering them, or both. More specifically, by chemical denaturing, it is meant that the chemical structure of an allergen containing protein is altered such that it no longer gives rise to allergic reactions in humans. While not wanting to be bound by any one theory, it is believed that the metal ions of the present compositions, and the optional allergen denaturing compounds, bind with the protein molecule in a way that prevents further reaction with a human's histadine. This is only one example of many potential chemical denaturing mechanisms. Ultimately, the allergic reaction is not initiated.

Likewise, physical "covering" of the allergen protein, or the particles such as dust, that contain these proteins, inhibit the initiation of the allergic reaction. While again, not wanting to be bound by any one theory, it is believed that an allergen protein must be solubilized or dispersed by the human body to initiate an allergic reaction. This can occur

when the protein contacts body fluids such as sweat on the skin, mucous in the eyes and nasal cavities, or saliva in the mouth. It is believed that if the protein is covered in a composition that does not readily solubilize or disperse in bodily fluids, the protein will not be available to initiate the allergic reaction. Moreover, physically covering a protein or the particles that contain the protein, for example, a house dust mite, the corpse of a house dust mite, or a common dust particle, the particles may be too heavy to become airborne. Because many allergens are inhaled, the simple act of "grounding" the allergen containing particles can significantly decrease the symptoms experienced by an allergy sufferer.

## I. ALLERGEN NEUTRALIZATION COMPOSITIONS

### *ALLERGEN NEUTRALIZING METAL IONS*

An essential component of the present invention is an allergen neuralizing metal ion, that is preferably supplied as a metallic salt. As discussed above, it is believed that these metal ions provide an allergen protein denaturing benefit. The metal ions are selected from the group consisting of ions of Zinc, Stannous, Stannic, Magnesium, Calcium, Manganese, Titanium, Iron, Manganese, Copper, Nickel, and mixtures thereof. Stannic, also known as $tin^{4+}$, is less preferred than stannous due primarily to solubility problems with stannic at low pH. But those skilled in the art will be able to formulate suitable compositions with either substance.

The preferred metal ions are zinc and stannous as these generally show high efficacy, with few detrimental environmental issues. Highly-ionized and soluble metal salts such as zinc chloride, provide the best source of metal ions. While certain cations yield superior solubility, all cations are acceptable for the metal salts of the present invention. For example, all halides, sulfates, ammonium, etc. Preferably the metallic salts are water-soluble zinc salts, stannous salts or mixtures thereof, , and especially $ZnCl_2$ and $SnCl_2$.

When metallic salts are added to the composition of the present invention they are typically present at a level of from about 0.01% to about 20%, preferably from about 0.1% to about 10%, more preferably from about 0.2% to about 8% by weight of the usage composition. When zinc salts are used as the metallic salt, and a clear solution is desired, it is preferable that the pH of the solution is adjusted to less than about 7, more preferably less than about 6, most preferably, less than about 5, in order to keep the solution clear.

### *ALLERGENDENATURING COMPOUNDS*

While the metal ions discussed above perform an allergen denaturing function, additional allergen denaturing compounds are incorporated in the present compositions. These allergen denaturing compounds are selected from the group consisting of polyphenol compounds, hydrogen peroxide, salicylic acid, citric acid, lactic acid, glycolic acid, ascorbic acid, gallic acid, gluconic acids, and mixtures thereof. Other alkyl acids are appropriate for use herein. When allergen denaturing compounds are incorporated into the compositions of this invention, it is preferred that low molecular weight alcohols, for example, ethanol, methanol, propanol or isopropanol, are used in the solvent. The solvent is discussed in greater detail below, but the use of low molecular weight alcohols in this context is to ensure solubility and stability of the allergen denaturing compounds in the composition. Low molecular weight alcohols are especially preferred when the concentration of the allergen denaturing compound exceeds 10% by weight of the allergen neutralization composition.

The polyphenol compounds include tannins, catechins, gallic acid and the like. These include either a natural or a synthetic substance such as, but not limited to, tannic acid or a synthetic tanning agent. Synthetic tannins/tannic acids generally fall into three chemical groups: 1) the so called auxiliary tans which are generally strong simple organic acids; 2) combination tans which are general sulphonic acids of complex phenolic materials; and, 3) exchange or replacement tans which are weakly acidic polymeric derivatives containing a large number of phenolic groups. Suitable tanning agents may be selected from cresol sulphonic acid ammounium salt (Neosyn RW), melamine formaldehyde sulphonate (Parnel A), a poly phenolic formaldehyde sulphonate (Suparex L) or a multiphenol formaldehyde sulphonate (Basyntan WL) as sold commercially for leather tanning or treatment. Other similar synthetic materials capable of reaction with protein (tanning action) may also prove effective when appropriately formulated. More recently formaldehyde reaction products of condensates and polymers of urea and melamine and mixtures thereof have been introduced for the manufacture of specialized leathers. These are also acceptable allergen denaturing compounds for the purpose of the present invention.

Naturally derived tannic acids and polyphenolics are also desirable for the present invention. The ones that do not develop color rapidly with air are most preferred. A most preferred tannin source is the kaki extract supplied as Pancil by Ririsu Scientific Industry of Osaka, Japan. This tannin is described in Japan Patent Application No 1991-3-61457; the entire disclosure of this Japan Patent Application is incorporated herein by reference.

When present, the allergen denaturing compositions are typically present at a level of from about 0.01% to about 20%, preferably from about 0.1% to about 10%, more preferably from about 0.2% to about 8% by weight of the usage composition.

***SOLVENT***

The solvent for the allergen neutralization compositions of the present invention preferably comprises water, and more preferably the solvent comprises a volatile lower alcohol. The water that is used can be distilled, deionized, or tap water. Water not only serves as the liquid carrier for the metal ions and other ingredients, it also facilitates the complexation reaction between the metal ion, the optional allergen denaturing compositions and the allergenic protein. Not to be bound by theory, it is believed that water solubilizes the allergenic protein allowing it to react with the metal ions, allergen denaturing compounds or both.

Low molecular weight alcohols with relatively low boiling points, as compared to water, such as methanol, ethanol, propanol and butanol, are preferred optional ingredients for improving the drying speed of the present compositions. Specifically, when the aqueous based compositions of this invention are sprayed on a solid surface, the surface necessarily becomes wet. Consumers, in general, do not like having wet counter tops, furniture, bedding and other common surfaces. Thus, for consumer acceptance, it is preferred that the compositions herein dry quickly after being applied to a surface. The addition of a low molecular weight alcohol substantially improves the drying time of the present compositions.

Typically, alcohol is added to the composition of the present invention at a level of from about 0.01% to about 20%, by weight of the composition, preferably from about 0.05% to about 10%, more preferably from about 0.1% to about 5.0%, by weight of the composition. It is understood that specialty products, for example, concentrated or refill solutions, solutions for industrial use, and the like, may contain higher levels of alcohol. In these products the alcohol can be present at levels of from greater than 20% to about 70%.

The solvent is preferably present in the allergen neutralization composition at a concentration of from 1% to 98%, preferably from about 3% to about 95%, and more preferably from about 5% to about 90%, by weight of the composition.

***WETTING AGENT***

It is preferred that the compositions of the present invention include a wetting agent such as a surfactant or the like. Most preferably, the wetting agent is fully compatible with the metal ions and the optional allergen denaturing compounds and other optional ingredients. The compatible wetting agent reduces surface tension of the composition of the present invention such that when the composition is sprayed on a surface the composition spreads evenly over the surface and wets the surface better. This allows the maximum amount of the active ingredients to contact the surface where the allergens may be present.

Preferred wetting agents for use in the present allergen neutralization compositions have an HLB of greater than about 8, and do not foam excessively. Excessive foaming can be determined by placing 300 ppm of the wetting agent in 100ml of distilled water in a 1000 ml stoppered graduated cylinder. The cylinder is shaken by inverting it 50 times. The cylinder should then be left to settle for 5 minutes. The resulting foam height after the settling period should be less than 50 ml. Non-limiting examples of wetting agents suitable for use herein include fatty alcohol ethloxylates, fatty alcohol ethoxylate-propoxylate, sulfates of alcohols or ethxoylated fatty alcohols, sorbitan monoesters, amine oxides, ethoxylated fatty acid esters, alkyl ether phosphates, alkyl polyglycosides, fatty acid glucosamides, alkyl phenol ethoxylates, alkyl phenol ethoxylated sulfates, paraffin sulfonates, fatty alcohols sulfates, alkyl phenyl sulfonates, linear alkyl benzene sulfonates, alkyl dimethyl betaines, alkyl dimethyl hydroxy propyl sultaines, alkyoxylated polydimethyl siloxanes, alkyl dimethyl amine oxides having alkyl chains with 6-18 carbons, and mixtures thereof In addition to the foregoing list, many other surfactant compounds selected from the group of anionic, nonionic, cationic, zwitterionic and mixtures thereof are suitable wetting agents for use herein. One preferred cationic surfactant suitable for use as a wetting agent in the present invention is Coco K3, which is a mono dodecanoate or tetradecanoate ester of methyl triethanol ammonium chloride.

Typical levels of wetting agents for use in the present compositions are from about 0.01% to about 3%, preferably from about 0.03% to about 2%, more preferably from about 0.05% to about 1.0%, by weight of the composition.

Nonlimiting examples of compatible nonionic surfactants include block copolymers of ethylene oxide and propylene oxide. Suitable block polyoxyethylenepolyoxypropylene polymeric surfactants, include those based on ethylene glycol, propylene glycol, glycerol, trimethylolpropane and ethylenediamine as the initial reactive hydrogen compound. Polymeric compounds made from a sequential ethoxylation and propoxylation of initial compounds with a single reactive hydrogen atom, such as $C_{12-18}$ aliphatic alcohols, are not generally compatible with the cyclodextrin. Certain of the block polymer surfactant compounds designated Pluronic® and Tetronic® by the BASF-Wyandotte Corp., Wyandotte, Michigan, are readily available. Examples of Pluronic® and Tetronic® surfactants are given below.

Nonlimiting examples of Pluronic Surfactants with the general formula $H(EO)_n(PO)_m(EO)_nH$, wherein EO is an ethylene oxide group, PO is a propylene oxide group, and n and m are numbers that indicate the average number of the groups in the surfactants. Typical examples of cyclodextrin-compatible Pluronic surfactants are:

| Name | Average MW | Average n | Average m |
|---|---|---|---|
| L-101 | 3,800 | 4 | 59 |
| L-81 | 2,750 | 3 | 42 |
| L-44 | 2,200 | 10 | 23 |
| L-43 | 1,850 | 6 | 22 |
| F-38 | 4,700 | 43 | 16 |
| P-84 | 4,200 | 19 | 43, |

and mixtures thereof.

Tetronic Surfactants have the general formula:

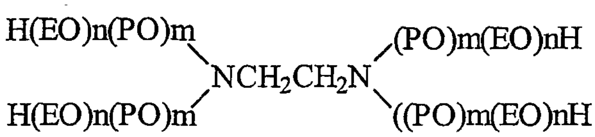

wherein EO, PO, n, and m have the same meanings as above. Typical examples of Tetronic surfactants are:

| Name | Average MW | Average n | Average m |
|---|---|---|---|
| 901 | 4,700 | 3 | 18 |
| 908 | 25,000 | 114 | 22, |

and mixtures thereof.

"Reverse" Pluronic and Tetronic surfactants have the following general formulas:

Reverse Pluronic Surfactants

$$H(PO)_m(EO)_n(PO)_mH$$

Reverse Tetronic Surfactants

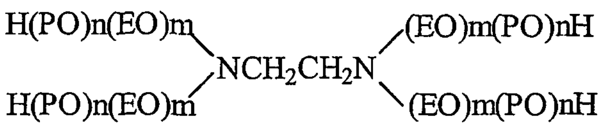

wherein EO, PO, n, and m have the same meanings as above. Typical examples of cyclodextrin-compatible Reverse Pluronic and Reverse Tetronic surfactants are:

Reverse Pluronic surfactants:

| Name | Average MW | Average n | Average m |
|---|---|---|---|
| 10 R5 | 1,950 | 8 | 22 |
| 25 R1 | 2,700 | 21 | 6 |

Reverse Tetronic surfactants

| Name | Average MW | Average n | Average m |
|---|---|---|---|
| 130 R2 | 7,740 | 9 | 26 |
| 70 R2 | 3,870 | 4 | 13 |

and mixtures thereof.

Another class of suitable nonionic wetting agents includes polyalkyleneoxide polysiloxanes having a dimethyl polysiloxane hydrophobic moiety and one or more hydrophilic polyalkylene side chains. Examples of this type of surfactants are the Silwet® surfactants which are available OSi Specialties, Inc., Danbury, Connecticut, and have the general formula:

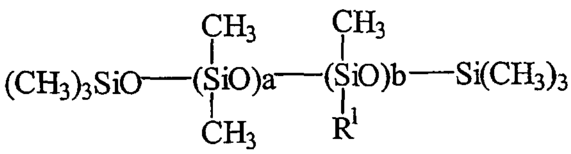

wherein a + b are from about 1 to about 50, preferably from about 3 to about 30, more preferably from about 10 to about 25, and $R^1$ is mainly one or more random poly(ethyleneoxide/propyleneoxide) copolymer groups having the general formula:

$$-(CH_2)_n\,O(C_2\,H_4\,O)_c\,(C_3\,H_6\,O)_d\,R^2$$

wherein n is 3 or 4, preferably 3; total c (for all polyalkyleneoxy side groups) has a value of from 1 to about 100, preferably from about 6 to about 100; total d is from 0 to about 14, preferably from 0 to about 3; and more preferably d is 0; total c+d has a value of from about 5 to about 150, preferably from about 9 to about 100 and each $R^2$ is the same or different and is selected from the group consisting of hydrogen, an alkyl having 1 to 4 carbon atoms, and an acetyl group, preferably hydrogen and methyl group.

Representative Silwet surfactants are as follows.

| Name | Average MW | Average a+b | Average total c |
|---|---|---|---|
| L-7608 | 600 | 1 | 9 |
| L-7607 | 1,000 | 2 | 17 |
| L-77 | 600 | 1 | 9 |
| L-7605 | 6,000 | 20 | 99 |
| L-7604 | 4,000 | 21 | 53 |
| L-7600 | 4,000 | 11 | 68 |
| L-7657 | 5,000 | 20 | 76 |
| L-7602 | 3,000 | 20 | 29 |

The molecular weight of the polyalkyleneoxy group ($R^1$) is less than or equal to about 10,000. Preferably, the molecular weight of the polyalkyleneoxy group is less than or equal to about 8,000, and most preferably ranges from about 300 to about 5,000. Thus, the values of c and d can be those numbers which provide molecular weights within these ranges. However, the number of ethyleneoxy units ($-C_2H_4O$) in the polyether chain ($R^1$) must be sufficient to render the polyalkyleneoxide polysiloxane water dispersible or water soluble. If propyleneoxy groups are present in the polyalkylenoxy chain, they can be distributed randomly in the chain or exist as blocks. Preferred Silwet surfactants are L-7600, L-7602, L-7604, L-7605, L-7657, and mixtures thereof. Besides surface activity, polyalkyleneoxide polysiloxane surfactants can also provide other benefits, such as antistatic benefits, lubricity and softness to fabrics.

The preparation of polyalkyleneoxide polysiloxanes is well known in the art. Polyalkyleneoxide polysiloxanes of the present invention can be prepared according to the procedure set forth in U.S. Pat. No. 3,299,112, incorporated herein by reference. Typically, polyalkyleneoxide polysiloxanes of the surfactant blend of the present invention are readily prepared by an addition reaction between a hydrosiloxane (i.e., a siloxane containing silicon-bonded hydrogen) and an alkenyl ether (e.g., a vinyl, allyl, or methallyl ether) of an alkoxy or hydroxy end-blocked polyalkylene oxide). The reaction conditions employed in addition reactions of this type are well known in the art and in general involve heating the reactants (e.g., at a temperature of from about 85° C. to 110° C.) in the presence of a platinum catalyst (e.g., chloroplatinic acid) and a solvent (e.g., toluene).

Nonlimiting examples of compatible anionic surfactants are the alkyldiphenyl oxide disulfonate, having the general formula:

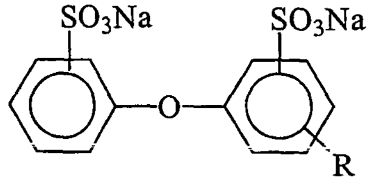

wherein R is an alkyl group. Examples of this type of surfactants are available from the Dow Chemical Company under the trade name Dowfax® wherein R is a linear or branched $C_6$-$C_{16}$ alkyl group. An example of these cyclodextrin-compatible anionic surfactant is Dowfax 3B2 with R being approximately a linear $C_{10}$ group. These anionic surfactants are preferably not used when the composition contains a cationic material so as to minimize the interaction with the cationic actives, since the effect of both surfactant and active are diminished.

***MITICIDES***

Miticides can be optionally added to the compositions of the present invention to kill mites. As is discussed above, mites corpses are allergenic so killing the mites does not necessarily reduce the level of allergens. But dead mites cannot breed, so killing a portion of the mites can help to control the mite population.

Miticides acceptable for use in the present invention include compounds known under the common names as resuethrin, phenothrin, permethrin, allethrins, tetramethrin, furamethrin, cypermethrin, decamethrin, phenvalerate, phenpropathrin, terallethrin, empenthrin and pyrethrin. Additional miticides include pyrethroid compounds such as 1-ethynyl-2-methyl-2-pentenyl-2,2-dimethyl-3-3-(2,2-dichlorovinyl)-cyclopropane-1-carboxylate, 1-ethynyl-2-methynyl-2-pentenyl-2,2,3,3-tetramethylcyclopropane-1-carboxylate, a-cyano-3-phenoxybenzyl-2,2-dimethyl-3-(2,2,3-tribromethyl)-cyclopropane-1-carboxylate; organic phosphorus compounds such as sumithion, fenthion, tetrachlorvinphos, diazinon and DDVP; and carbamate compounds such as those sold under the trademarks Baygon and Sevin.

A number of less toxic miticidal agents have been proposed for use in controlling dust mites. As noted in U.S. Patent No. 4,800,196, these include phenyl salicylate, diphenylamine, methyl β-naphthyl ketone, coumarin, phenethyl benzoate, benzyl salicylate, phenyl benzoate, N-fluorodichloromethylthio-cyclohexene-dicarboxyimide, p-nitrobenzoic acid methyl ester, p-chlorometaxylenol, α-bromocinnamic aldehyde, 2,5-dichloro-4-bromophenol, N,N-dimethyl-N'-tryl-N'-(fluorodichloromethylthio)-sulfamide, 2-phenylphenol, sodium 2-phenylphenolate, 5-chloro-2-methyl-4-isothiazoline-3-one, 2-methyl-4-isothiazonoline-3-one and benzimidazolylmethyl-carbamate and mixtures of these. One of the more effective agents for killing dust mites is benzyl benzoate, a compound which is readily available and inexpensive.

When one or more optional miticides are added to the composition of the present invention they are typically present at a level of from about 0.01% to about 20%, preferably from about 0.1% to about 10%, more preferably from about 0.2% to about 8% by weight of the usage composition.

***COLOR STABILIZATION INGREDIENTS***

The allergen neutralization compositions can optionally include ingredients to prevent color formation either in the product or on the fabrics and articles on which it is sprayed. As mentioned above, some of the allergen denaturing compounds, especially the preferred tannins, are known to discolor fabric materials. The optional color stabilization ingredients are intended to reduce or eliminate the discoloration problem. When used, the color stabilization ingredient will be present at a concentration of from about 0.1% to about 25%, preferably from about 0.5% to about 15% and more preferably from about 1% to about 10%, by weight of the allergen neutralization composition. Preferred chemicals include glycolic acid and its salts, lactic acid and its salts, gluconic acid and its salts, pyruvic acid and its salts, glucaric acid and its salts, ascorbic acid and its salts, hydroxy benzoic acids and the salts, aspartic acid and its salts, hydroxyglutamic acid and its salts, hydroxyphathalic acids and the salts, malic acid and its salts, and mixtures thereof.

Soluble detergent builders can also be included for color stabilization. Included among the soluble polycarboxylate builders are a variety of categories of useful materials. One important category of polycarboxylate builders encompasses the ether polycarboxylates, including oxydisuccinate, as disclosed in Berg, U.S. 3,128,287, U.S. 3,635,830. See also "TMS/TDS" builders of U.S. 4,663,071. Suitable ether polycarboxylates also include cyclic compounds, particularly alicyclic compounds, such as those described in U.S. 3,923,679; 3,835,163; 4,158,635; 4,120,874 and 4,102,903.

Other useful builders include the ether hydroxypolycarboxylates, copolymers of maleic anhydride with ethylene or vinyl methyl ether, 1, 3, 5-trihydroxy benzene-2, 4, 6-trisulphonic acid, and carboxymethyloxysuccinic acid, the various salts of polyacetic acids such as ethylenediamine tetraacetic acid and nitrilotriacetic acid, as well as polycarboxylates such as mellitic acid, pyromellitic, succinic acid, oxydisuccinic acid, polymaleic acid, benzene 1,3,5-tricarboxylic acid,

carboxymethyloxysuccinic acid, and soluble salts thereof.

Citrate builders, e.g., citric acid and soluble salts thereof (particularly sodium salt), are polycarboxylate builders of particular importance due to their availability from renewable resources and their biodegradability. Oxydisuccinates are also especially useful in such compositions and combinations. Other suitable polycarboxylates are disclosed in U.S 4,144,226 and in U.S. 3,308,067. See also U.S. 3,723,322.

Also suitable in the compositions of the present invention are the 3,3-dicarboxy-4-oxa-1,6-hexanedioates and the related compounds disclosed in U.S. 4,566,984. Useful succinic acid builders include the $C_5$-$C_{20}$ alkyl and alkenyl succinic acids and salts thereof. A particularly preferred compound of this type is dodecenylsuccinic acid. Specific examples of succinate builders include: laurylsuccinate, myristylsuccinate, palmitylsuccinate, 2-dodecenylsuccinate (preferred), 2-pentadecenylsuccinate, and the like. Laurylsuccinates are the preferred builders of this group, and are described in EP 0,200,263.

Fatty acids, e.g., $C_{12}$-$C_{18}$ monocarboxylic acids such as oleic acid and/or its salts, can also be incorporated into the compositions alone, or in combination with the aforesaid builders, especially citrate and/or the succinate builders, to provide additional builder activity. Such use of fatty acids will generally result in a diminution of foaming, which should be taken into account by the formulator.

In situations where phosphorus-based builders can be used, the various alkali metal phosphates such as the well-known sodium tripolyphosphates, sodium pyrophosphate and sodium orthophosphate can be used. Phosphonate builders such as ethane-1-hydroxy-1,1-diphosphonate and other known phosphonates (see, for example, U.S. Patents 3,159,581; 3,213,030; 3,422,021; 3,400,148 and 3,422,137) can also be used.

***PERFUME***

The allergen neutralization compositions of the present invention can also optionally include a perfume to provide a pleasing scent to the spray product. The perfume should not be designed to be overwhelming or to be used as an odor masking ingredient. Perfumes are typically added at low levels, e.g., from about 0% to about 0.5%, preferably from about 0.003% to about 0.3%, more preferably from about 0.005% to about 0.2%, by weight of the usage composition.

Perfume can also be added as a more intense odor in product and on surfaces. When stronger levels of perfume are preferred, relatively higher levels of perfume can be added. Any type of perfume can be incorporated into the composition of the present invention. Preferably the perfume is hydrophilic and is composed predominantly of ingredients selected from two groups of ingredients, namely, (a) hydrophilic ingredients having a ClogP of less than about 3.5, more preferably less than about 3.0, and (b) ingredients having significant low detection threshold, and mixtures thereof. Typically, at least about 50%, preferably at least about 60%, more preferably at least about 70%, and most preferably at least about 80% by weight of the perfume is composed of perfume ingredients of the above groups (a) and (b). Suitable perfume ingredients can be found in US Patent No. 5,670,475, which issued to Trinh et al. On September 23, 1997. The entire disclosure of the Trinh patent is incorporated herein by reference.

***OTHER OPTIONAL INGREDIENTS***

The compositions of the present invention can optionally contain ingredients including, solution phase stabilizers, chelating agents, antistatic agents, colorants, especially bluing agents, antioxidants, and mixtures thereof. The total level of optional ingredients is low, preferably less than about 8.0%, more preferably less than about 5.0%, and even more preferably less than about 3.0%, by weight of the usage composition. Solution phase stabilizers include, alkali metal salts, for example, NaCl, $CaCl_2$, $MgCl_2$, KCl, $KSO_4$, and they are added to modify viscosity as well as to stabilize the solution. These optional ingredients exclude the other ingredients specifically mentioned hereinbefore.

**Colorant**

Colorants and dyes, especially bluing agents, can be optionally added to the present compositions for visual appeal and performance impression. When colorants are used, they are used at extremely low levels to avoid fabric staining. Preferred colorants for use in the present compositions are highly water-soluble dyes, e.g., Liquitint® dyes available from Milliken Chemical Co. Non-limiting examples of suitable dyes are, Liquitint Blue HP®, Liquitint Blue 65®, Liquitint Patent Blue®, Liquitint Royal Blue®, Liquitint Experimental Yellow 8949-43®, Liquitint Green HMC®, Liquitint Yellow II®, and mixtures thereof, preferably Liquitint Blue HP®, Liquitint Blue 65®, Liquitint Patent Blue®, Liquitint Royal Blue®, Liquitint Experimental Yellow 8949-43®, and mixtures thereof.

**Optional Preservative**

Optionally, but preferably, solubilized, water-soluble, antimicrobial preservative can be added to the compositions. Because microbial growth in aqueous solutions is highly objectionable when it occurs, it is highly preferable to include a solubilized, water-soluble, antimicrobial preservative, which is effective for inhibiting or regulating microbial growth in order to increase storage stability of the preferably clear, aqueous allergen neutralization compositions of this invention.

It is preferable to use a broad spectrum preservative, e.g., one that is effective on both bacteria (both gram positive and gram negative) and fungi. A limited spectrum preservative, e.g., one that is only effective on a single group of microorganisms, e.g., fungi, can be used in combination with a broad spectrum preservative or other limited spectrum preservatives with complimentary and/or supplementary activity. A mixture of broad spectrum preservatives can also be used. In some cases where a specific group of microbial contaminants is problematic (such as Gram negatives), aminocarboxylate chelators may be used alone or as potentiators in conjunction with other preservatives. These chelators, which include, e.g., ethylenediaminetetraacetic acid (EDTA), hydroxyethylenediaminetriacetic acid, diethylenetriaminepentaacetic acid, and other aminocarboxylate chelators, and mixtures thereof, and their salts, and mixtures thereof, can increase preservative effectiveness against Gram-negative bacteria, especially *Pseudomonas* species.

Antimicrobial preservatives useful in the present invention include biocidal compounds, i.e., substances that kill microorganisms, or biostatic compounds, i.e., substances that inhibit and/or regulate the growth of microorganisms.

Preferred antimicrobial preservatives are those that are water-soluble and are effective at low levels. Water-soluble preservatives useful in the present invention are those that have a solubility in water of at least about 0.3 g per 100 ml of water, i.e., greater than about 0.3% at room temperature, preferably greater than about 0.5% at room temperature.

The water-soluble antimicrobial preservative in the present invention is included at an effective amount. The term "effective amount" as herein defined means a level sufficient to prevent spoilage, or prevent growth of inadvertently added microorganisms, for a specific period of time. In other words, the preservative is not being used to kill microorganisms on the surface onto which the composition is deposited in order to eliminate odors produced by microorganisms. Instead, it is preferably being used to prevent spoilage of the allergen neutralization composition in order to increase the shelf-life of the composition. Preferred levels of preservative are from about 0.0001% to about 0.5%, more preferably from about 0.0002% to about 0.2%, most preferably from about 0.0003% to about 0.1%, by weight of the usage composition.

The preservative can be any organic preservative material which will not cause damage to fabric appearance, e.g., discoloration, coloration, bleaching. Preferred water-soluble preservatives include organic sulfur compounds, halogenated compounds, cyclic organic nitrogen compounds, low molecular weight aldehydes, quaternary ammonium compounds, dehydroacetic acid, phenyl and phenolic compounds, and mixtures thereof.

**Anti-Oxidant**

The compositions of the present invention can optionally comprise anti-oxidants. The level of anti-oxidants can vary widely depending upon the end use of the composition. When present, the compositions will typically comprise from about 0.01% to about 10%, more typically from about 0.1% to about 5%, by weight, of anti-oxidants.

Preferred anti-oxidants herein are selected from the group consisting of D-isoascorbic acid, Dibutyl hydroxy toluene (BHT), dl-alpha-tocophenol, n-dodecyl gallate, styrenated phenol, 4, 4'-butylidene bis (6-ter-butyl-3-methylpheno), 4,4'-thio bis (6-tert-butyl-3-methylphenol), 1,1-bis (4-hydroxyphenyl)cyclohexane, 2,2,thio (diethyl bis 3,3,5-di-t-butyl-4-hydroxyphenyl) propionate, hexane-1,6-diamine, N, N'-bis (-2,2,6,6,-tetramethyl-4-diperidinyl), and mixture therof.

**II. ARTICLE OF MANUFACTURE**

The compositions of the present invention can also be used in an article of manufacture comprising said composition plus a spray dispenser. When the commercial embodiment of the article of manufacture is used, it is optional, but preferable, to include the preservative. Therefore, the most basic article of manufacture comprises a metal ion, a solvent, and a spray dispenser.

***SPRAY DISPENSER***

The article of manufacture herein comprises a spray dispenser. The allergen neutralization composition is placed into a spray dispenser in order to be distributed onto fabrics or other surfaces. The spray dispenser is preferably any of the manually activated means for producing a spray of liquid droplets as is known in the art, e.g. trigger-type, pump-type, non-aerosol self-pressurized, and aerosol-type spray means. The spray dispenser herein does not normally include those that will substantially foam the allergen neutralization composition. It has been found that the performance is increased by providing smaller particle droplets. Desirably, the Sauter mean particle diameter is from about 10 μm to

about 120 μm, more preferably, from about 20 μm to about 100 μm.

The spray dispenser can be an aerosol dispenser. An aerosol dispenser comprises a container which can be constructed of any of the conventional materials employed in fabricating aerosol containers. The dispenser must be capable of withstanding internal pressure in the range of from about 20 to about 110 p.s.i.g., more preferably from about 20 to about 70 p.s.i.g. One important requirement concerning the dispenser is that it be provided with a valve member which will permit the allergen neutralization composition contained in the dispenser to be dispensed in the form of a spray of very fine, or finely divided, particles or droplets. The aerosol dispenser utilizes a pressurized sealed container from which the allergen neutralization composition is dispensed through a special actuator/valve assembly under pressure. The aerosol dispenser is pressurized by incorporating therein a gaseous component generally known as a propellant. Common aerosol propellants, e.g., gaseous hydrocarbons such as isobutane, and mixed halogenated hydrocarbons, which are not preferred. Halogenated hydrocarbon propellants such as chlorofluoro hydrocarbons have been alleged to contribute to environmental problems. Preferred propellants are compressed air, nitrogen, inert gases, carbon dioxide, etc. A more complete description of commercially available aerosol-spray dispensers appears in U.S. Pat. Nos.: 3,436,772, Stebbins, issued April 8, 1969; and 3,600,325, Kaufman et al., issued August 17, 1971; both of said references are incorporated herein by reference.

Preferably the spray dispenser can be a self-pressurized non-aerosol container having a convoluted liner and an elastomeric sleeve. Said self-pressurized dispenser comprises a liner/sleeve assembly containing a thin, flexible radially expandable convoluted plastic liner of from about 0.010 to about 0.020 inch thick, inside an essentially cylindrical elastomeric sleeve. The liner/sleeve is capable of holding a substantial quantity of odor-absorbing fluid product and of causing said product to be dispensed. A more complete description of self-pressurized spray dispensers can be found in U.S. Pat. Nos. 5,111,971, Winer, issued May 12, 1992, and 5,232,126, Winer, issued Aug. 3, 1993; both of said references are herein incorporated by reference. Another type of aerosol spray dispenser is one wherein a barrier separates the odor absorbing composition from the propellant (preferably compressed air or nitrogen), as disclosed in U.S. Pat. No. 4,260,110, issued April 7, 1981, and incorporated herein by reference. Such a dispenser is available from EP Spray Systems, East Hanover, New Jersey.

More preferably, the spray dispenser is a non-aerosol, manually activated, pump-spray dispenser. Said pump-spray dispenser comprises a container and a pump mechanism which securely screws or snaps onto the container. The container comprises a vessel for containing the aqueous odor-absorbing composition to be dispensed.

The pump mechanism comprises a pump chamber of substantially fixed volume, having an opening at the inner end thereof. Within the pump chamber is located a pump stem having a piston on the end thereof disposed for reciprocal motion in the pump chamber. The pump stem has a passageway there through with a dispensing outlet at the outer end of the passageway and an axial inlet port located inwardly thereof.

The container and the pump mechanism can be constructed of any conventional material employed in fabricating pump-spray dispensers, including, but not limited to: polyethylene; polypropylene; polyethyleneterephthalate; blends of polyethylene, vinyl acetate, and rubber elastomer. A preferred container is made of clear, e.g., polyethylene terephthalate. Other materials can include stainless steel. A more complete disclosure of commercially available dispensing devices appears in: U.S. Pat. Nos.: 4,895,279, Schultz, issued January 23, 1990; 4,735,347, Schultz et al., issued April 5, 1988; and 4,274,560, Carter, issued June 23, 1981; all of said references are herein incorporated by reference.

Most preferably, the spray dispenser is a manually activated trigger-spray dispenser. Said trigger-spray dispenser comprises a container and a trigger both of which can be constructed of any of the conventional material employed in fabricating trigger-spray dispensers, including, but not limited to: polyethylene; polypropylene; polyacetal; polycarbonate; polyethyleneterephthalate; polyvinyl chloride; polystyrene; blends of polyethylene, vinyl acetate, and rubber elastomer. Other materials can include stainless steel and glass. A preferred container is made of clear, e.g. polyethylene terephthalate. The trigger-spray dispenser does not incorporate a propellant gas into the odor-absorbing composition, and preferably it does not include those that will foam the odor-absorbing composition. The trigger-spray dispenser herein is typically one which acts upon a discrete amount of the odor-absorbing composition itself, typically by means of a piston or a collapsing bellows that displaces the composition through a nozzle to create a spray of thin liquid. The trigger-spray dispenser typically comprises a pump chamber having either a piston or bellows which is movable through a limited stroke response to the trigger for varying the volume of said pump chamber. This pump chamber or bellows chamber collects and holds the product for dispensing. The trigger spray dispenser typically has an outlet check valve for blocking communication and flow of fluid through the nozzle and is responsive to the pressure inside the chamber. For the piston type trigger sprayers, as the trigger is compressed, it acts on the fluid in the chamber and the spring, increasing the pressure on the fluid. For the bellows spray dispenser, as the bellows is compressed, the pressure increases on the fluid. The increase in fluid pressure in either trigger spray dispenser acts to open the top outlet check valve. The top valve allows the product to be forced through the swirl chamber and out the nozzle to form a discharge pattern. An adjustable nozzle cap can be used to vary the pattern of the fluid dispensed.

For the piston spray dispenser, as the trigger is released, the spring acts on the piston to return it to its original position. For the bellows spray dispenser, the bellows acts as the spring to return to its original position. This action

causes a vacuum in the chamber. The responding fluid acts to close the outlet valve while opening the inlet valve drawing product up to the chamber from the reservoir.

A more complete disclosure of commercially available dispensing devices appears in U.S. Pat. Nos. 4,082,223, Nozawa, issued Apr. 4, 1978; 4,161, 288, McKinney, issued Jul. 17, 1985; 4,434,917, Saito et al., issued Mar. 6, 1984; and 4,819,835, Tasaki, issued Apr. 11, 1989; 5,303,867, Peterson, issued Apr. 19, 1994; all of said references are incorporated herein by reference.

A broad array of trigger sprayers or finger pump sprayers are suitable for use with the compositions of this invention. These are readily available from suppliers such as Calmar, Inc., City of Industry, California; CSI (Continental Sprayers, Inc.), St. Peters, Missouri; Berry Plastics Corp., Evansville, Indiana, a distributor of Guala® sprayers; or Seaquest Dispensing, Cary, Illinois.

The preferred trigger sprayers are the blue inserted Guala® sprayer, available from Berry Plastics Corp., or the Calmar TS800-1A® , TS1300®, and TS-800-2®, available from Calmar Inc., because of the fine uniform spray characteristics, spray volume, and pattern size. More preferred are sprayers with precompression features and finer spray characteristics and even distribution, such as Yoshino sprayers from Japan. Any suitable bottle or container can be used with the trigger sprayer, the preferred bottle is a 17 fl-oz. bottle (about 500 ml) of good ergonomics similar in shape to the Cinch® bottle. It can be made of any materials such as high density polyethylene, polypropylene, polyvinyl chloride, polystyrene, polyethylene terephthalate, glass, or any other material that forms bottles. Preferably, it is made of high density polyethylene or clear polyethylene terephthalate.

For smaller fluid ounce sizes ( such as 1 to 8 ounces), a finger pump can be used with canister or cylindrical bottle. The preferred pump for this application is the cylindrical Euromist II® from Seaquest Dispensing. More preferred are those with precompression features.

### III. METHOD OF USE

The present compositions can be used by distributing, e.g., by placing the allergen neutralizing composition into a dispensing means, preferably a spray dispenser and spraying an effective amount onto the desired surface or article. An effective amount is defined herein as an amount to neutralize at least about 50%, preferably at least about 60%, more preferably at least about 80%, and most preferably at least about 90% of the allergens on the surface or article that is sprayed. The amount of allergen that is neutralized can be measured by the Elisa test defined below. The delivery mechanism should be controlled such that a pool of liquid is not created on the article or surface and so that when dry there is no visual deposit readily discernible. Distribution can be achieved by using a spray device, a roller, a pad, etc.

The present invention encompasses the method of spraying an effective amount of allergen neutralizing composition onto household surfaces. Preferably the household surfaces are selected from the group consisting of countertops, cabinets, walls, floors, bathroom surfaces and kitchen surfaces.

The present invention encompasses the method of spraying a mist of an effective amount of allergen neutralizing composition onto fabric and/or fabric articles. Preferably, the fabric and/or fabric articles include, but are not limited to, clothes, curtains, drapes, upholstered furniture, carpeting, bed linens, bath linens, tablecloths, sleeping bags, tents, car interior, etc.

The present invention relates to the method of spraying a mist of an effective amount of allergen neutralizing composition onto cat litter, pet bedding and pet houses.

### EXAMPLES

The following are non-limiting examples of allergen neutralization compositions according to the present invention, and standardized methods for determining the efficacy and fabric coloration of the present compositions.

### EXAMPLE I

#### Efficacy Determination Protocol, "ELISA"

The efficacy of an allergen neutralization composition can be determined by the ELISA protocol. Enzyme-linked immunosorbent assay ("ELISA") is a highly sensitive assay technique for detection and measurement of antigens or antibodies in solution. The assay uses enzyme-linked antigens or antibodies to amplify an antigen-antibody reaction. A single enzyme-linked antibody-antigen complex can convert orders of magnitude more of colorless substrate molecules into detectable colored products, thus considerably amplifying the original antigen-antibody reaction. The antigen or antibody is adsorbed onto the surface of the well of a microtiter plate, and all the relevant reactions take place in solution inside the well. While the following protocol will be easily understood by those skilled in the art, for a more detailed

explanation please see Konishi, E. and Uehara, K. Enzyme-linked immunosorbent assay for quantifying antigens of *Dermatophagoides farinae* and *D. pteronyssinus* (Acari: Pyroglyphidae) in house dust samples, 1990, *Entomologist Society of America,* **27** (6), p. 993-998.

It is understood that those skilled in the art of allergen suppression will be aware of numerous variations to the protocol given below. For example, the monoclonal antibody may be substituted with a similar antibody, for example Der f 1 for Der f 2. But a standard, in this case water, is used and the results for the samples that contain active ingredients are normalized against the standard and the results reported as percentage of allergen remaining That is, the amount of allergen that is not neutralized. Thus, the allergen neutralization results for similar, but not identical antibodies should be substantially the same when normalized to the standard. Both Der f 1 and Der f 2 were tested for various compositions, but Der f 2 was chosen as the standard for the present Efficacy Determination Protocol because it is more difficult to neutralize. To be clear, the values for the % of allergen remaining that are reported in Table I will generally improve (that is, decrease) when tested with Der f 1.

### *Sample Preparation*

At least two samples are prepared. One sample contains only distilled water and samples of one or more allergen neutralization compositions containing the allergen neutralization active, for example ZnCl2, tannin, etc. The samples are all treated according to the same protocol, and the distilled water sample is used as the control. All samples are prepared as follows:

1) Add 1.0 ml of 20ug/ml Der f 2 mite extract to 0.8 ml of the sample composition (either the allergen neutralization composition or water for the control). Mix well and let sit for 1 hour at room temp.
2) Add 2.2 ml of 20% skim milk in Phosphate Buffer Saline (PBS).
3) Mix well by vortexing and centrifuge at 12000 rpm (approx. 17000xg) for 1 hour.
4) The supernatant is used for the ELISA protocol.

### *ELISA Protocol*

Each well of a microplate is coated at 4°C overnight with 100 μl of Der f 2 monoclonal antibody (clone 15E11, from Asahi Breweries., AB-A-4) at a protein concentration of 2 μg per ml of Phosphate Buffer Saline (PBS), pH 7.4 containing 0.1% sodium azide. The monoclonal antibody is discarded and the plate is incubated with 100 μl of 1% BSA PBS containing 0.1% sodium azide for 1 hour. The plate is washed 3 times with PBS containing 0.05% Tween 20 (PBS-T). 100 μl of an allergen sample is added and incubated for 1 hour at 37°C.

Use 3x dilutions of a reference Dermatophagoides farinae crude extract (commercially available from LSL) to make the standard curve. The curve is made using 6.9 nanogram-mite extract equivalent/ml to 5 microgram-mite extract equivalent/ml. Wash plate with PBS-T for 3 times. 100 μl of Horse Radish Peroxidase (HRP) conjugated anti Der f 2 antibody (clone 13A4) is added at 40 ng/ml and the plate is incubated at 37°C for 1 hour. The plate is then washed with PBS-T 3 times and then washed with water 3 times. The wells are incubated with 100 μl of o-phenylene diamine at 2.0 mg/ml of 0.1M phosphate buffer (pH 6.2) containing 0.03% $H_2O_2$. The enzyme reaction is terminated by adding 50 μl of 2 M $H_2SO_4$.

The absorbance of the sample is read at 490 nm with a cell length of 1 cm. The absorbance reading is directly proportional to the quantity of Der f 2 bound and the values calculated from the respective standard curve (Optical Density, or absorbance range is 0.001 to 3.6). The absorbance reading for the control (water) is considered as 0% allergen neutralization (100% of the Der f 2 remaining) and an absorbance reading of 0 is considered 100% allergen neutralization (0% of the Der f 2 remaining). The absorbance of the active containg sample(s) is then normalized based on these two end points.

As stated above, the allergen neutralization compositions of the present invention neutralize at least about 50%, preferably at least about 60%, more preferably at least about 80% and most preferably at least about 90% of allergen containing proteins as measured by the ELISA test protocol.

Table I illustrates that the "effective amount" of the metal ion will vary depending on the metal and its associated anion. The concentration of 1.4% was an arbitrary selection, but one can see that except for $FeSO_4$, all of the metals remove more than 50% of the allergen. Thus, the effective amount to remove 50% of allergens for $FeSO_4$ is greater than 1.4%, while the effective amount for the same removal rate for $SnCl_2$ is substantially less than 1.4%. Moreover, the effective amount will vary based on the desired allergen removal amount. For example, if 80% removal is necessary, then the effective amount of $ZnCl_2$ will be slightly greater than 1.4%, and the effective amount for $SnCl_2$ will be about 1.4%. Those skilled in the art will be able to determine the effective amount for different metal ions with various anions without undue experimentation using the teachings and Examples herein.

**TABLE I**

| Active Compound | Wt. % of Ion* | pH | Average Absorbance | % of Allergen Remaining |
|---|---|---|---|---|
| Control | 0 | - | 1.7 | 100 |
| $ZnCl_2$ | 1.4 | 3.6 | 0.70 | 34 |
| $SnCl_2$ | 1.4 | 2.1 | 0.54 | 32 |
| $SnCl_2$ | 1.4 | 3.2 | 0.25 | 18 |
| $SnSO_4$ | 1.4 | 2.0 | 0.28 | 22 |
| $FeSO_4$ | 1.4 | 3.5 | 1.13 | 54 |
| $FeCl_2$ | 1.4 | 2.9 | 0.84 | 44 |
| *% by weight of the active ion in the allergen neutralization composition before the Sample Preparation. | | | | |

## EXAMPLE II

### Fabric Color Evaluation Method

A standard Hunter Color Meter Analysis is performed on fabrics that are treated with sample allergen neutralization compositions. As was described above, it is desirable that the present compositions do not stain or discolor common household surfaces such as fabrics, for example, bed covers, floor coverings, and curtains, and hard surfaces, for example, counter tops, furniture, doors and walls. A standardized test is given below that tests the propensity for a given sample composition to stain a test fabric. Acceptable performance for this test method will generally mean a composition can be used on household surfaces without fear of substantial discoloration.

As stated above, the allergen neutralization compositions of the present invention yield a $\Delta E$ value of less than about 1.0, preferably less than 0.95 and more preferably less than about 0.90, as measured by Fabric Color Evaluation method.

### *Sample Preparation*

Spray approximately 15 grams of a sample composition on a 20x20 cm cotton knit (100% cotton) fabric swatch. Spray approximately 15 grams of distilled water on a similar 20x20 cm fabric swatch. Dry the fabric swatches near a glass-window to expose them to sun light. Leave the swatches near the window for 2-3 days after they have dried. The sun light accelerates the color development. The treated and non-treated fabric swatches are ready for analysis by the method defined below.

### *Instrument*

The instrument used in this method is a ColorQUEST 45/0 from Hunter Association Laboratory Inc., Virginia (USA). The light source is D65.

### *Method*

The "L", "a", and "b" values for each fabric swatch are measured at three different places. L, a, and b values are measured both for the treated fabric swatches and the control fabric swatch. "L" measures lightness and varies from 100 (for perfect white) to zero (for black); "a" measures the intensity of red (plus), gray (zero) and green (minus); "b" measures the intensity of yellow (plus), gray (zero) and blue (minus). $\Delta E$ is the difference of "L a b" values from the standard. The Hunter Lab total color difference ($\Delta E$) is calculated as follows:

$$\Delta E = \sqrt{\Delta L^2 + \Delta a^2 + \Delta b^2}$$

where: $\Delta L = L_{SMP} - L_{STD}$; $\Delta a = a_{SMP} - a_{STD}$; $\Delta b = b_{SMP} - b_{STD}$.

***Sample Measurements***

Two solutions containing Tannic acid and Zinc chloride are prepared and a third solution containing only Zinc chloride is prepared. The concentration of Tannic acid (from Kanto Chemicals, Japan) is 0.025%, 0.05% and 0%, by weight in the three solutions. The concentration of Zinc chloride (from Wako Pure Chemicals, Japan) is 0.5%, by weight in all three samples. The three samples are each split into two pairs of six equal samples. The pH of each solution is adjusted to the desired pH of about 3.6. One sample from each pair is adjusted with 110mM citrate buffer (citric acid and sodium citrate), and one sample from each pair is adjusted with 0.4mM HCl, as shown in the table below. Six sample swatches are prepared according to the method given above using the six different compositions. The colors for each swatch, and a control swatch, are measured and the results are shown in the following table:

**TABLE II**

| Tannic acid (%) | ZnCl2 (%) | pH adjusted to 3.6 with | L | a | b | ΔE |
|---|---|---|---|---|---|---|
| Standard (No treatment) | | | 94.67 | -0.19 | 1.18 | - |
| 0.025 | 0.5 | HCl | 92.22 | -0.13 | 3.82 | 3.61 |
| 0.05 | 0.5 | HCl | 91.74 | -0.21 | 4.21 | 4.22 |
| 0.025 | 0.5 | citrate | 94.87 | -0.17 | 1.18 | 0.21 |
| 0.05 | 0.5 | citrate | 94.58 | -0.17 | 1.24 | 0.15 |
| 0.0 | 0.5 | HCL | | | | <0.1 |
| 0.0 | 0.5 | citrate | | | | <0.1 |

In this test, the Citrate buffer (110 mM) prevents color formation on fabrics treated with a combination of tannic acid (at least up to 0.05%) and Zinc ion. It should be noted that compositions containing no tannin create very little color formation regardless of the buffer solution.

## EXAMPLE III

Table III contains sample compositions according to the present invention.

**TABLE III A**

| Material | 1 Wt. % | 2 Wt. % | 3 Wt. % | 4 Wt. % | 5 Wt. % |
|---|---|---|---|---|---|
| $ZnCl_2$ | 3.0 | 2.0 | 0 | 0 | 2.0 |
| $ZnSO_4$ | 0 | 0 | 3.0 | 5.0 | 2.0 |
| Tannin | 0 | 0.4 | 0.5 | 0 | 1.0 |
| Buffer* | 0.05 | 0.2 | 0.15 | 0.1 | 0.2 |
| Diethylene Glycol** | 0.4 | 0.5 | 0.8 | 0.2 | 0 |
| Wetting agent*** | 0.05 | 0.1 | 0.5 | 0.1 | 0.1 |
| Ethanol | 3.0 | 5.0 | 10.0 | 3.0 | 0 |
| Water | Balance | Balance | Balance | Balance | Balance |

**TABLE III B**

| Material | 6 Wt. % | 7 Wt. % | 8 Wt.% | 9 Wt. % | 10 Wt. % |
|---|---|---|---|---|---|
| $SnCl_2$ | 4.0 | 2.0 | 0 | 0 | 1.5 |
| $SnSO_4$ | 0 | 0 | 3.0 | 4.0 | 2.0 |
| Tannin | 0 | 0.4 | 0.5 | 0 | 1.0 |
| Buffer* | 0.05 | 0.2 | 0.15 | 0.1 | 0.2 |

(continued)

| Material | 6 Wt. % | 7 Wt. % | 8 Wt.% | 9 Wt. % | 10 Wt. % |
|---|---|---|---|---|---|
| Diethylene Glycol** | 0.4 | 0.5 | 0.8 | 0.2 | 0 |
| Wetting agent*** | 0.05 | 0.1 | 0.5 | 0.1 | 0.1 |
| $NaCl_2$ | 0 | 3.0 | 0 | 0 | 3.0 |
| Ethanol | 3.0 | 5.0 | 10.0 | 3.0 | 0 |
| Water | Balance | Balance | Balance | Balance | Balance |
| *Buffer is HCl, Citric acid, or mixtures of each<br>**Diethylene Glycol is used to neutralize the odor associated with ethanol<br>***Wetting agent is Silwet L-7600 or Coco K3 (a cationic surfactant) | | | | | |

## Claims

1. An allergen neutralization composition for use on inanimate objects, the composition comprising:

   an effective amount of an allergy neutralizing metal ion;
   a solvent; and
   an allergen denaturing compound,

   wherein the allergen neutralization composition is sprayable,
   and further wherein the metal ion is selected from the group consisting of ions of zinc, stannous, stannic, magnesium, calcium, manganese, titanium, iron, copper, nickel, and mixtures thereof, preferably the metal ion is selected from the group consisting of zinc, stannous and mixtures thereof, and the allergen denaturing compound is selected from the group consisting of polyphenol compounds, hydrogen peroxide, salicylic acid, citric acid, lactic acid, glycolic acid, ascorbic acid, gallic acid, gluconic acids, and mixtures thereof.

2. The allergen neutralization composition of claim 1, wherein the composition neutralizes at least about 50%, preferably at least about 60%, more preferably at least about 80% and most preferably at least about 90% of allergen containing proteins as measured by the ELISA test protocol.

3. The allergen neutralization composition of claim 1, further comprising a wetting agent.

4. The allergen neutralization composition of claim 1, wherein the composition is essentially free of cyclodextrin based complexing agents.

5. The allergen neutralization composition of claim 1, wherein the solvent comprises water.

6. The allergen neutralization composition of claim 1, wherein the solvent comprises from about 0.01% to about 20%, preferably from about 0.05% to about 10%, more preferably from about 0.1% to about 5.0%, by weight of the composition of a volatile lower alcohol.

7. The allergen neutralization composition of claim 1, wherein the metal ion is present in the composition at about 0.01% to about 20%, preferably from about 0.1% to about 10%, more preferably from about 0.2% to about 8%, by weight, of the solution.

8. The allergen neutralization composition of claim 1, further comprising at least about 0.05% by weight of an allergen neutralizing polyphenol compound, wherein the allergen neutralization composition yields a $\Delta E$ value of less than about 1.0 as measured by Fabric Color Evaluation method.

9. The allergen neutralization composition of claim 1, further comprising a miticide.

## Patentansprüche

1. Allergenneutralisationszusammensetzung zum Gebrauch auf unbelebten Gegenständen, wobei die Zusammensetzung Folgendes umfasst:

   eine wirksame Menge an einem allergieneutralisierenden Metallion;
   ein Lösungsmittel; und
   eine allergendenaturierende Verbindung,
   wobei die Allergenneutralisationszusammensetzung sprühfähig ist,
   und wobei ferner das Metallion ausgewählt ist aus der Gruppe, bestehend aus Ionen von Zink, Zinn(II), Zinn (IV), Magnesium, Calcium, Mangan, Titan, Eisen, Kupfer, Nickel und Mischungen davon, das Metallion vorzugsweise ausgewählt ist aus der Gruppe, bestehend aus Zink, Zinn(II) und Mischungen davon, und die allergendenaturierende Verbindung ausgewählt ist aus der Gruppe, bestehend aus Polyphenolverbindungen, Wasserstoffperoxid, Salicylsäure, Citronensäure, Milchsäure, Glycolsäure, Ascorbinsäure, Gallussäure, Gluconsäuren und Mischungen davon.

2. Allergenneutralisationszusammensetzung nach Anspruch 1, wobei die Zusammensetzung mindestens ungefähr 50 %, vorzugsweise mindestens ungefähr 60 %, mehr bevorzugt mindestens ungefähr 80 % und am meisten bevorzugt mindestens ungefähr 90 % der allergenhaltigen Proteine, wie anhand des ELISA-Testprotokolls gemessen, neutralisiert.

3. Allergenneutralisationszusammensetzung nach Anspruch 1, die ferner ein Benetzungsmittel umfasst.

4. Allergenneutralisationszusammensetzung nach Anspruch 1, wobei die Zusammensetzung im Wesentlichen frei von Komplexierungsmitteln auf Cyclodextrinbasis ist.

5. Allergenneutralisationszusammensetzung nach Anspruch 1, wobei das Lösungsmittel Wasser umfasst.

6. Allergenneutralisationszusammensetzung nach Anspruch 1, wobei das Lösungsmittel von ungefähr 0,01 Gew.-% bis ungefähr 20 Gew.-%, vorzugsweise von ungefähr 0,05 Gew.-% bis ungefähr 10 Gew.-%, mehr bevorzugt von ungefähr 0,1 Gew.-% bis ungefähr 5,0 Gew.-% der Zusammensetzung einen flüchtigen niederen Alkohol umfasst.

7. Allergenneutralisationszusammensetzung nach Anspruch 1, wobei das Metallion in der Zusammensetzung zu ungefähr 0,01 Gew.-% bis ungefähr 20 Gew.-%, vorzugsweise von ungefähr 0,1 Gew.-% bis ungefähr 10 Gew.-%, mehr bevorzugt von ungefähr 0,2 Gew.-% bis ungefähr 8 Gew.-% der Lösung vorliegt.

8. Allergenneutralisationszusammensetzung nach Anspruch 1, die ferner zu mindestens ungefähr 0,05 Gew.-% eine allergenneutralisierende Polyphenolverbindung umfasst, wobei die Allergenneutralisationszusammensetzung einen $\Delta$E-Wert von weniger als ungefähr 1,0, wie anhand des Stofffarbbewertungsverfahrens gemessen, ergibt.

9. Allergenneutralisationszusammensetzung nach Anspruch 1, die ferner ein Milbenabtötungsmittel umfasst.

## Revendications

1. Composition de neutralisation d'allergène pour utilisation sur des objets inanimés, la composition comprenant :

   une quantité efficace d'un ion métallique de neutralisation d'allergie ;
   un solvant ; et
   un composé de dénaturation d'allergène,
   où la composition de neutralisation d'allergène est vaporisable,
   et, en outre, dans laquelle l'ion métallique est choisi dans le groupe constitué d'ions de zinc, stanneux, stanniques, de magnésium, de calcium, de manganèse, de titane, de fer, de cuivre, de nickel, et leurs mélanges, de préférence l'ion métallique est choisi dans le groupe constitué de zinc, stanneux et leurs mélanges, et le composé de dénaturation d'allergène est choisi dans le groupe constitué de composés de polyphénol, de peroxyde d'hydrogène, d'acide salicylique, d'acide citrique, d'acide lactique, d'acide glycolique, d'acide ascorbique, d'acide gallique, d'acides gluconiques, et leurs mélanges.

2. Composition de neutralisation d'allergène selon la revendication 1, où la composition neutralise au moins environ 50 %, de préférence au moins environ 60 %, plus préférablement au moins environ 80 % et le plus préférablement au moins environ 90 % de protéines contenant un allergène, comme mesuré par le protocole de test ELISA.

3. Composition de neutralisation d'allergène selon la revendication 1, comprenant, en outre, un agent mouillant.

4. Composition de neutralisation d'allergène selon la revendication 1, où la composition est pratiquement exempte d'agents complexants à base de cyclodextrine.

5. Composition de neutralisation d'allergène selon la revendication 1, dans laquelle le solvant comprend de l'eau.

6. Composition de neutralisation d'allergène selon la revendication 1, dans laquelle le solvant comprend d'environ 0,01 % à environ 20 %, de préférence d'environ 0,05 % à environ 10 %, plus préférablement d'environ 0,1 % à environ 5,0 %, en poids de la composition d'un alcool inférieur volatil.

7. Composition de neutralisation d'allergène selon la revendication 1, dans laquelle l'ion métallique est présent dans la composition à un taux d'environ 0,01 % à environ 20 %, de préférence d'environ 0,1 % à environ 10 %, plus préférablement d'environ 0,2 % à environ 8 %, en poids, de la solution.

8. Composition de neutralisation d'allergène selon la revendication 1, comprenant, en outre, au moins environ 0,05 % en poids d'un composé polyphénol de neutralisation d'allergène, où la composition de neutralisation d'allergène donne une valeur $\Delta E$ inférieure à environ 1,0, comme mesuré par le procédé Fabric Color Evaluation.

9. Composition de neutralisation d'allergène selon la revendication 1, comprenant, en outre, un acaricide.